# DEMANDE DE BREVET EUROPEEN

(11) **EP 0 866 334 A1**
(43) Date de publication de la demande: **23.09.1998**
(21) Numéro de dépôt: 98400550.4
(22) Date de dépôt: 09.03.1998
(51) Int. Cl.: G01N 33/497

(54) **Dispositif échantillonneur, notamment pour analyseur de gaz**

(30) Priorité: 18.03.1997 FR 9703294
(71) Demandeur: PLASTO S.A., 21300 Chenôve (FR); RENAULT, 92109 Boulogne-Billancourt (FR)
(72) Inventeur: Le Poivre, Evelyne, 91440 Bures-sur-Yvette (FR); Tavernier, Laurent, 21000 Dijon (FR); Planque, Sophie, 75017 Paris (FR); Czajkowski, Leszek, 21000 Dijon (FR)
(74) Mandataire: Levy, David

(57) **Abrégé**

Il est du type comprenant une chambre de prélèvement (5) à volume variable en communication avec une cellule d'analyse et de mesure (11), des moyens d'admission (8) dudit échantillon de fluide dans ladite chambre de prélèvement (5) et des moyens de transfert (7, 12, 14-21) dudit échantillon de fluide de la chambre de prélèvement vers la cellule d'analyse et de mesure et il est caractérisé en ce que les moyens de transfert (7, 12, 14-21) sont déplacés avec une vitesse contrôlée pour balayer la cellule d'analyse et de mesure (11) avec un débit prédéterminé d'échantillon.

## Description

La présente invention concerne un dispositif échantillonneur, notamment adapté aux appareils de contrôle électronique des gaz, permettant de faire circuler le gaz avec un débit constant dans la chambre de mesure.

### Art antérieur

Le contrôle analytique des gaz peut faire appel à différentes techniques suivant lesquelles l'échantillon devra être introduit dans l'appareil soit de façon ponctuelle sur un laps de temps le plus court possible -cas par exemple d'un chromatographe- soit avec un débit régulier et pendant une durée de temps suffisante -cas par exemple d'un capteur particulier tel qu'une électrode spécifique.

Ce second cas, qui nous intéresse plus particulièrement dans le cadre de la présente invention, nécessite généralement que le capteur spécifique atteigne un état d'équilibre pour fournir une mesure stable et fiable. Pour obtenir ce résultat, il est important que l'interface entre le capteur et l'élément gazeux soit homogène et dans un état stationnaire, soit immobile, soit en déplacement régulier, constant et faible. Pour obtenir de telles conditions dans le cas de la mesure d'un gaz, on peut utiliser par exemple une seringue motorisée ou un système mécanique de pompage à débit continu ou encore des systèmes mettant en oeuvre un capteur de pression, une soupape, une temporisation comme décrit par exemple dans FR-A-2 449 877. Ces systèmes sont généralement complexes ou nécessitent des dispositifs trop encombrants pour être installés dans un appareil portable et léger tel qu'un éthylotest.

### Objet de l'invention

La présente invention a pour but de fournir un dispositif simple et autonome permettant l'échantillonnage d'un gaz qui doit être analysé par un capteur spécifique. Un tel dispositif trouve son application notamment dans les éthylotests, utilisés généralement par les conducteurs de véhicules lorsqu'ils veulent s'assurer du respect de la réglementation concernant l'alcoolémie au volant.

### Description

Le dispositif selon l'invention comprend une chambre à volume variable définie par un cylindre dans lequel se déplace un piston sous l'effet d'un jeu de ressorts armés manuellement, les mouvements du piston étant contrôlés dans leur vitesse par un ralentisseur et dans leur cycle par un système de levier à action fin de course.

Ce système, purement mécanique, permet d'assurer les cycles nécessaires à l'échantillonnage et au balayage de la cellule de mesure de l'appareil qui est alimentée à débit constant par le gaz à analyser.

### Description détaillée

D'autres avantages et caractéristiques apparaîtront clairement à la lecture de la description d'un mode de réalisation préféré de l'invention, ainsi que des dessins annexés sur lesquels :
- la figure 1 est une vue d'ensemble en perspective éclatée d'un dispositif selon l'invention,
- la figure 2 est une représentation schématique des moyens de liaison et de transfert entre la chambre de prélèvement d'échantillon et la chambre d'analyse et de mesure,
- les figures 3 à 5 sont des vues de dessins partiellement en coupe d'une partie du dispositif représenté sur la figure 1.

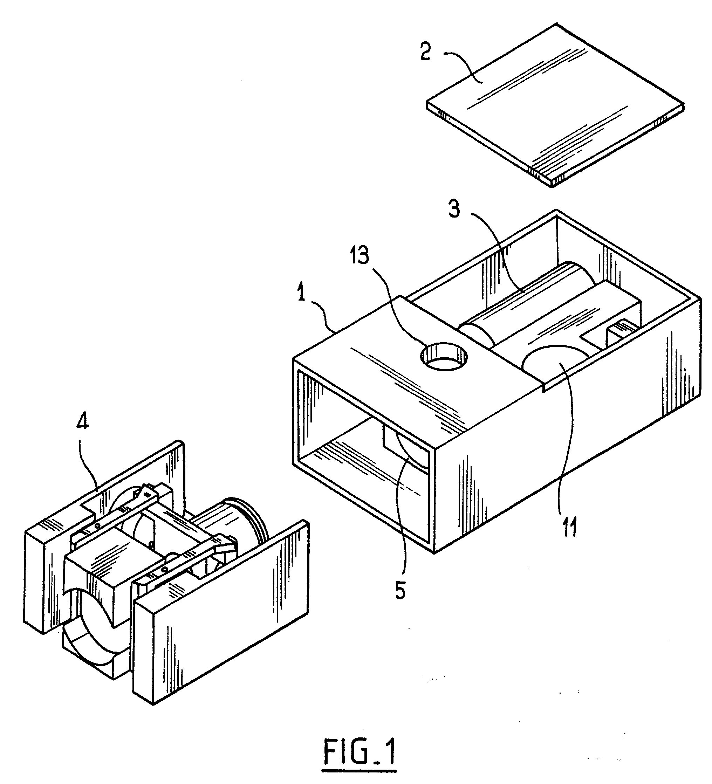

Le dispositif selon l'invention, tel que représenté sur la figure 1, comprend un boîtier 1 dans lequel est disposé un circuit imprimé 2 qui assure les différentes fonctions électroniques nécessaires au fonctionnement du dispositif, l'alimentation en puissance électrique étant assurée par une ou plusieurs piles électriques 3. Un ensemble tiroir 4 qui sera décrit ultérieurement est également logé dans le boîtier 1 et coopère avec d'autres éléments prévus dans ledit boîtier 1.

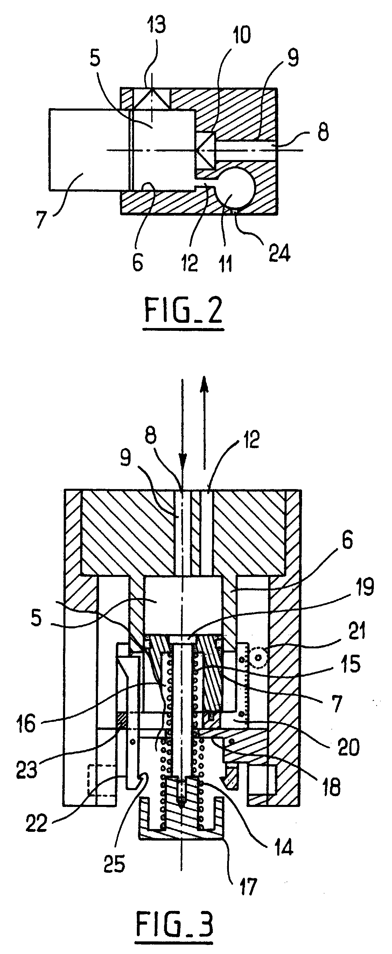

La figure 2 représente le dispositif échantillonneur 4 dans son état de repos. Le dispositif comprend une chambre de prélèvement d'échantillon 5 définie par un cylindre 6 et un piston 7. La chambre de prélèvement ou d'échantillonnage 5 est pourvue d'un orifice d'entrée 8 qui communique avec une source d'un fluide (non représentée) dont on veut analyser et mesurer un des composants, ledit fluide étant par exemple l'air expiré par un individu et le composant étant par exemple l'alcool alvéolaire contenu dans l'air expiré.

Dans la conduite 9 reliant la chambre d'échantillonnage 4 à l'entrée 8 est monté un clapet anti-retour 10, ladite chambre d'échantillonnage 5 étant reliée par une conduite 12 à une chambre d'analyse 11 comprenant un capteur approprié tel qu'un capteur d'éthanol. Un autre clapet anti-retour 13 est monté dans la chambre d'échantillonnage et fonctionne en valve d'échappement. Les deux clapets 10 et 13 sont constitués chacun par exemple par une valve magnétique et ont pour fonction de retenir l'air alvéolaire expiré, une fois l'expiration terminée. De plus, ces valves sont calculées de manière à s'ouvrir sous un seuil de pression déterminé, obligeant l'utilisateur à souffler avec au moins ce minimum de pression.

Le mécanisme de commande du mouvement du piston 7 comprend un bouton poussoir 17 et un jeu de deux ressorts 14 et 15 montés sur un axe 16 coaxial au piston 7. Le premier ressort 14 travaille en compression et il est en appui par une de ses extrémités sur le bouton poussoir 17 et par son autre extrémité sur le châssis 18 de l'ensemble support du cylindre ; le second ressort 15 travaille également en compression et il prend appui par une de ses extrémités sur le bouton poussoir 17 et par son autre extrémité sur le fond du piston 7. L'axe 16 qui traverse le piston est pourvu d'un épaulement 19 apte à ramener le piston 7 à son point mort bas correspondant au volume maximum de la chambre d'échantillonnage 5. Un étrier 20, solidaire du piston 7, coopère d'une part avec un système ralentisseur 21 et d'autre part avec un levier 22 par le moyen d'un index 23 fixé sur l'étrier 20.

Comme indiqué précédemment, la figure 3 représente un dispositif dans son état de repos, la chambre d'échantillonnage 5 étant dans son état de volume maximum. Lorsque le dispositif se trouve dans cet état, on emplit la chambre par le gaz à analyser ; ainsi par exemple, lorsque le dispositif est un élément d'un éthylotest, l'utilisateur souffle dans l'entrée 8, par l'intermédiaire d'un embout buccal, non représenté, de façon à bien balayer la chambre d'échantillonnage 5 avec l'air expulsé des poumons : l'air en excès est évacué à travers la valve d'échappement 13 et par la chambre d'analyse et de mesure 11 qui communique avec l'atmosphère par l'intermédiaire d'un orifice d'évacuation 24 (figure 2). A ce stade, l'échantillon de gaz contenu dans la chambre d'échantillonnage 5 est considéré comme représentatif du gaz à analyser.

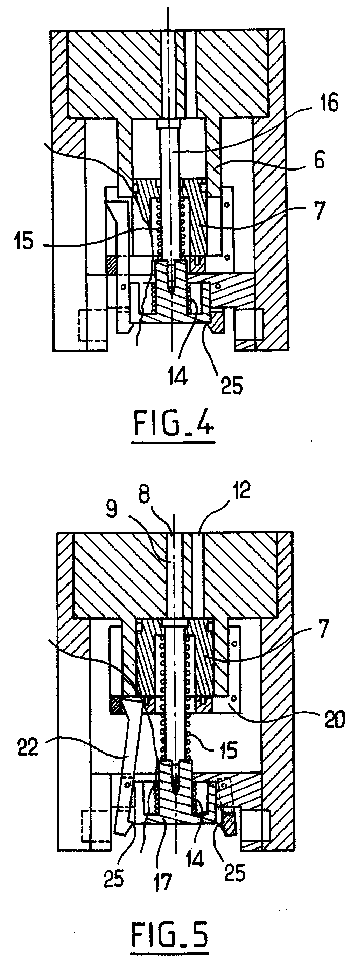

On effectue alors une pression manuelle sur le bouton poussoir 17, qui a pour effet de comprimer les ressorts 14 et 15 et de verrouiller le bouton poussoir 17 sous une encoche 25 ménagée sur le levier 22, ainsi que cela est représenté sur la figure 4. L'action combinée du ressort 14, qui pousse le piston 7 vers son point mort haut et du ralentisseur 21 qui freine le déplacement du piston 7 permet d'obtenir un mouvement lent du piston 7 vers son point mort haut avec pour conséquence une évacuation du gaz de la chambre d'échantillonnage 5 vers la chambre d'analyse du capteur 11 avec un débit pratiquement constant : cette période pendant laquelle le capteur reçoit le gaz échantillonné à débit régulier est mise à profit pour effectuer la mesure et fournir un résultat fiable et reproductible.

Lorsque le piston 7 arrive en fin de course à son point mort haut qui correspond au volume minimum de la chambre d'échantillonnage 5, l'index 23 solidaire de l'étrier 20 bascule le levier 22 et libère le bouton poussoir 17 ; cet état est représenté par la figure 5. Le ressort 14, qui se trouve toujours dans un état compressé, repousse le bouton poussoir 17 et entraîne simultanément l'axe 16 et le piston 7 vers leur position initiale, pour atteindre l'état de volume maximal de la chambre d'échantillonnage 5. Le retour du piston 7, freiné par le ralentisseur 21, provoque une aspiration d'air frais à travers l'orifice 24 et la chambre d'analyse 11 qui se trouve être balayée en sens inverse du flux au moment de l'analyse du gaz. Ce retour à la position initiale ramène ainsi le capteur dans un environnement de gaz neutre, propice pour effectuer la mesure dite "zéro" d'étalonnage du capteur.

On voit ainsi que, à partir du moment où la chambre d'échantillonnage a été remplie du gaz à analyser, une seule poussée sur le bouton poussoir permet de dérouler le cycle nécessaire à l'analyse et que ce dernier se déroule de façon reproductible indépendamment de la force avec laquelle le bouton poussoir a été manoeuvré. Un tel dispositif, extrêmement simple peut être facilement synchronisé avec la partie électronique du capteur et ainsi utilisable sans difficulté par des personnes non spécialisées dans le domaine de l'analyse. L'utilisation de ce dispositif d'échantillonnage pour un éthylotest portable permet de disposer d'un appareil fiable et simple d'emploi.

La réalisation du dispositif fait appel à des techniques et matériaux couramment utilisés en micro-mécanique ; pour la fabrication des différents composants, à l'exception des ressorts, on peut utiliser avantageusement les matières plastiques telles que par exemple le Nylon ou l'ABS. Les clapets sont de préférence du type à membrane à maintien magnétique, le clapet mobile étant fabriqué à base d'un matériau ferromagnétique, et maintenu dans sa position fermée par un aimant situé dans le siège. Le dispositif ralentisseur est choisi parmi les appareils connus comme par exemple des ralentisseurs en rotation par friction grasse (commercialisés par exemple par les sociétés COMITRONIC ou ITW de France) ou des micro-vérins (par exemple commercialisés par CLIPPARD).

Le dispositif peut éventuellement être complété pour un système de temporisation asservi à un capteur de pression afin de garantir un balayage optimisé de la chambre d'échantillonnage. Le perfectionnement, non indispensable au fonctionnement du dispositif, simplifie encore l'utilisation et peut augmenter la fiabilité du résultat obtenue si l'utilisateur n'est pas familier des appareils d'analyse.

## Revendications

1. Dispositif portable de prélèvement et d'analyse d'au moins un échantillon d'un fluide, du type comprenant une chambre de prélèvement (5) à volume variable en communication avec une cellule d'analyse et de mesure (11), des moyens d'admission (8) dudit échantillon de fluide dans ladite chambre de prélèvement (5) et des moyens de transfert (7, 12, 14-21) dudit échantillon de fluide de la chambre de prélèvement vers la cellule d'analyse et de mesure, lesdits moyens de transfert comprenant un piston (7) se déplaçant à vitesse contrôlée, caractérisé en ce que ledit piston (7) se déplace sous l'effet d'un jeu de ressorts (14, 15) armés manuellement et d'un ralentisseur (21), pour balayer la cellule d'analyse et de mesure (11) avec un débit prédéterminé d'échantillon.

2. Dispositif selon la revendication 1, caractérisé en ce que la cellule d'analyse et de mesure (11) est balayée pendant l'analyse et la mesure avec un débit d'échantillon constant.

3. Dispositif selon la revendication 1 ou 2, du type dans lequel la chambre de prélèvement (5) est définie par un cylindre (6) et un piston (7) mobile dans ledit cylindre, caractérisé en ce que le mouvement du piston est commandé par au moins deux ressorts (14,15) montés coaxialement sur un axe (16) disposé coaxialement dans ledit piston (7) et solidaire d'un poussoir d'actionnement (17).

4. Dispositif selon la revendication 3, caractérisé en ce qu'un des ressorts (14) prend appui par ses extrémités sur le piston (7) et le poussoir d'actionnement (17), l'autre ressort (15) prenant appui par ses extrémités sur le bouton poussoir (17) et un organe fixe solidaire (18) du cylindre, les deux ressorts étant mis en compression.

5. Dispositif selon l'une quelconque des revendications 1 à 4, caractérisé en ce qu'il comprend des moyens ralentisseurs (21) du mouvement du piston.

6. Dispositif selon l'une quelconque des revendications 1 à 5, caractérisé en ce qu'il comprend, en outre, des moyens (19) de rappel complémentaires du piston (7) dans une position correspondant au volume maximum de la chambre de prélèvement (5).

7. Dispositif selon l'une quelconque des revendications 1 à 6, caractérisé en ce qu'il comprend, en outre, un étrier (20) monté sur ledit piston et coopérant avec des moyens ralentisseurs (21) et un levier (22) de verrouillage et de déverrouillage du poussoir d'actionnement (17).

8. Dispositif selon l'une quelconque des revendications 1 à 7, caractérisé en ce qu'il comprend un circuit électronique (2) relié à un capteur de mesure disposé dans la cellule d'analyse et de mesure (11).
